# EUROPÄISCHE PATENTSCHRIFT

(11) **EP 2 163 220 B1**
(45) Veröffentlichungstag und Bekanntmachung des Hinweises auf die Patenterteilung: **01.10.2014**
(21) Anmeldenummer: 09011600.5
(22) Anmeldetag: 10.09.2009
(51) Int. Cl.: A61C 1/08, A61B 17/17

(54) **Zahnmedizinisches Bohrführungssystem**
Dental drilling system
Système de guidage de forage médical dentaire

(30) Priorität: 10.09.2008 DE 202008012049 U
(43) Veröffentlichungstag der Anmeldung: 17.03.2010
(73) Patentinhaber: Gäßler Zahntechnik GmbH, 89079 Ulm (DE)
(72) Erfinder: Gäßler, Guido, 89081 Ulm (DE)
(74) Vertreter: Otten, Herbert

(56) Entgegenhaltungen:
- WO-A-03/071972
- WO-A-2007/079775
- FR-A- 2 896 403
- FR-A- 2 910 804
- FR-A- 2 916 626
- US-A- 5 743 916
- US-A1- 2008 220 390

## Beschreibung

Die Erfindung betrifft ein zahnmedizinisches Bohrführungssystem für eine zahnmedizinische Implantat-Behandlung nach dem Oberbegriff des Schutzanspruchs 1.

### Stand der Technik

Es werden allgemein zahnmedizinische Implantate für den Aufbau eines einzelnen, zu ersetzenden Zahnkörpers bzw. einer Anordnung von mehreren Zahnkörpern verwendet. Dabei wird ein so genanntes Implantatbefestigungselement in den Kiefer eingebracht, welches mit einem entsprechenden Ansatzelement über die Kieferschleimhaut hinausragt. Dieses Ansatzelement wird für den späteren Aufbau bzw. für die Befestigung eines Zahnkörpers verwendet.

Mit der Druckschrift DE 10 2007 015 818 A1 wird eine Anordnung zur Einbringung von Implantaten gezeigt, insbesondere Dentalimplantaten, wobei eine Schiene eine Bohrung aufweist, welche zur Herstellung einer Kieferbohrung in einem für das Implantat vorgesehenen Bereich ausgebildet ist. Die Führungsschiene bildet eine ortsgenaue Anordnung des Implantats aus. Ferner umfasst die Anordnung ein Eindrehwerkzeug für das Implantat, welches ein Kopfteil aufweist, mit welchem das Implantat verbunden ist und welches eine mit der Führungsfläche in Eingriff bringbare Außenfläche aufweist. Das Eindrehwerkzeug weist ferner einen ersten Anschlag auf, wodurch die Führungsschiene im Bereich der Bohrung einen weiteren Anschlag zumindest mittelbar ausbildet, an welchem der erste Anschlag des Eindrehwerkzeugs beim Eindrehen des Implantats zur Vorgabe dessen Einbringtiefe zur Anlage gelangt.

Dieser vorbenannten Druckschrift ist zu entnehmen, dass in die Ausnehmung der Schiene eine Führungshülse sowie eine Bohrhülse angeordnet werden, um den erforderlichen Dentalbohrer zur Herstellung einer Kieferbohrung zu führen.

Aus der US 5,743,916 ist ein Bohrführungssystem mit austauschbaren Führungen bekannt, welche in eine Außenhülse abwechselnd entsprechend dem zu führenden Bohrerdurchmesser einsetzbar sind.

Aus der WO 03/071972 ist ein Führungssystem mit variablen Abstandshülsen zur Tiefenbegrenzung bekannt.

Aus den Dokumenten FR 2 910 804, FR 2 896 403 sowie WO 2007/079775 A1 sind ebenfalls Bohrführungssysteme bekannt, welche mittels einzelner Führungsmittel eine korrekte Bohrposition bereitstellen sollen.

Nachteil dieses Führungssystems ist, dass die Bohrhülse keine Arretierung in Rotationsrichtung des Bohrers gegenüber der Führungshülse aufweist, was zu Störungen bei der Herstellung der Kieferbohrung führen kann bzw. die Flexibilität des Anwenders einschränkt.

Zudem erfordert die Anordnung einen entsprechenden Platzbedarf zur Herstellung einer Kieferbohrung, da der Bohrer in einer nahezu senkrechten Anordnung mit Bezug auf den darunter liegenden Kiefer geführt werden soll.

Aus diesem Grunde hat sich die Erfindung die Aufgabe gestellt, ein Führungssystem zur Herstellung einer zahnmedizinischen Bohrung derart weiter zu bilden, dass eine flexible und kostengünstige Implantat-Behandlung ausgeführt werden kann.

Zur Lösung der Aufgabe ist die Erfindung durch die Merkmale des Schutzanspruchs 1 gekennzeichnet.

Vorteilhafte Erweiterungen der Erfindung sind in den abhängigen Schutzansprüchen beschrieben.

### Offenbarung der Erfindung

Die Neuerung geht von einem zahnmedizinischen Bohrführungssystem für eine zahnmedizinische Implantat-Behandlung gemäß Anspruch 1 aus.

Die Ausbildung der neuerungsgemäßen Führungsvorrichtung hat den Vorteil, dass während der zahnmedizinischen Implantat-Behandlung durch den Einsatz von ineinander steckbaren, separaten Führungshülsen die Herstellung einer Kieferbohrung mit unterschiedlichen Bohrerdurchmessern durchführbar ist. Hierdurch kann die Bohrung schrittweise vergrößert werden, ohne dass zu viel Reibungswärme erzeugt wird.

Darüber hinaus gewährleistet die Haltevorrichtung zur Aufnahme der Führungsvorrichtung eine flexible Anordnung eines Bohrführungssystems im Mundhöhlenbereich eines Patienten.

In einer bevorzugten Ausgestaltung der Neuerung ist vorgesehen, dass die mindestens zwei ineinander steckbaren Führungshülsen lösbar miteinander verbunden sind.

Die lösbare Verbindung der ineinander steckbaren Führungshülsen bildet den Vorteil aus, dass der Einsatz von einer Vielzahl von Dentalbohrern mit unterschiedlichen Durchmessern möglich ist, da der Innendurchmesser des jeweiligen Fräs- bzw. Bohrwerkzeugs angepasst werden kann.

Darüber hinaus gewährleisten die ineinander steckbaren Führungshülsen einen flexiblen Einsatz von unterschiedlichen Bohr- bzw. Fräswerkzeugen.

Die hier verwendete lösbare Verbindung verhindert darüber hinaus ein Mitdrehen von den ineinander gesteckten Führungshülsen während der Herstellung einer Kieferbohrung.

In einer über dies bevorzugten Ausgestaltung der Neuerung ist vorgesehen, dass die Verbindung der Führungshülsen mittels einer auf der Manteloberfläche jeder inneren Führungshülse teilweise angeordneten, radial nach außen gerichteten Auswölbung ausgebildet wird.

Eine Verbindung mittels einer Auswölbung gewährleistet eine einfache und kostengünstige Verbindungsform zwischen zwei rotationssymmetrischen Teilen aus, wobei die nach außen gerichtete Auswölbung auf der Manteloberfläche einer inneren Führungshülse einer entsprechenden radial nach außen gerichteten Auswölbung auf der Innenoberfläche einer äußeren Führungshülse zugeordnet ist.

Dadurch wird eine Verbindungstechnik zwischen zwei Führungshülsen gebildet, die als eine leicht verriegelbare bzw. leicht lösbare Verbindung ausgebildet werden kann. Überdies wird in einer weiteren Ausgestaltung der Neuerung bevorzugt, dass die im direkten Kontakt mit der Haltevorrichtung stehende Führungshülse auf ihrer inneren Mantelfläche eine Ausnehmung aufweist.

Diese Ausnehmung ist zur Aufnahme einer Auswölbung auf der Mantelfläche einer ersten inneren Führungshülse vorgesehen, wobei die Manteloberfläche der im direkten Kontakt mit der Haltevorrichtung stehenden, äußeren Führungshülse vorzugsweise frei von Auswölbungen auf ihrer äußeren Manteloberfläche ist, da aufgrund eines direkten Kontaktes mit der Haltevorrichtung, beispielsweise durch eine Zementverbindung, eine zusätzliche Verbindung nicht erforderlich ist.

Vorzugsweise können auf der äußeren Mantelfläche Bereiche vorgesehen sein, welche beispielsweise in Form von ebenen oder rauen Flächen einen verbesserten Halt in der Haltevorrichtung bereitstellen.

In einer weiteren, bevorzugten Ausgestaltung der Neuerung ist vorgesehen, dass die radial nach außen gerichtete Auswölbung einer inneren Führungshülse in eine Ausnehmung in der Mantelfläche einer die innere Führungshülse aufnehmende äußere Führungshülse mittels Formschluss der Auswölbung in der Ausnehmung eingreift.

Dieser Formschluss wird mittels Verdrehen der beiden Führungshülsen gegeneinander ausgeführt, vorzugsweise in der Art eines Bajonettverschlusses. Greifen die beiden Auswölbungen der beiden ineinander gesteckten und gegeneinander verdrehten Führungshülsen ineinander ein, wird ein Formschluss gebildet, der ein weiteres Verdrehen der inneren Führungshülse in Rotationsrichtung des Fräs- bzw. Bohrwerkzeuges sowie ein axiales Verschieben der zwei ineinander gesteckten Führungshülsen vermeidet.

Die Verbindungstechnik in der Art eines Bajonett-Verschlusses kann insbesondere noch dadurch in ihrer Handhabung vereinfacht werden, dass die Ausnehmung bzw. die Auswölbung mit einem stumpfen, insbesondere an ebenen Ausnehmungsenden in Mantelumlaufrichtung ausgebildeten Anschlag enden. Diese stumpf ausgebildeten Enden der Verbindung verhindern ein nachhaltiges Verklemmen aufgrund von ineinandertreibenden Drehmomenten bei der Führung eines Werkzeugs. Keilförmige Ausnehmungen können ineinander verklemmen, ebene aufeinander treffende Flächen der Verbindung nach Art des Bajonettverschlusses eigen diesen Effekt nicht.

Es ist vorgesehen, dass die Mantelfläche jeder Führungshülse in axialer Richtung teilweise durchbrochen ist.
Diese Ausnehmung in axialer Richtung gewährleistet ein störungsfreies Einfügen von zwei ineinander zu steckenden Führungshülsen, da beim Ineinanderstecken von zwei Führungshülsen der Bereich der Auswölbung auf der Manteloberfläche der inneren Führungshülse in den axial durchbrochenen Bereich der äußeren Führungshülse eingeführt werden kann.
Darüber hinaus ist es aufgrund dieser Durchbrechung möglich, dass ein Bohr- bzw. Fräswerkzeug im hinteren Kieferbereich seitlich, vorzugsweise mit einem schrägen Verlauf, in die Führungshülse eingeführt werden kann. Dadurch verringert sich der Platzbedarf zur Benutzung eines neuerungsgemäßen Bohrführungssystems vorteilhaft.

Ein zusätzlicher Vorteil der neuerungsgemäßen Durchbrechung ist das störungsfreie Abführen von Bohr- bzw. Frässpäne über die am Werkzeug angeordnete Wendelnut bei einer Bohrtiefe bis zum Tiefenanschlag des Werkzeuges, Dadurch wird ein zusätzliches Erwärmen der sensiblen Körperstrukturen aufgrund entstehender Reibungswärme vermieden.

In einer weiteren Ausgestaltung der Neuerung ist vorgesehen, dass die innere Führungshülse einen Innendurchmesser aufweist, welcher einem Außendurchmesser eines zu führenden Bohr- bzw. Fräswerkzeugs angepasst ist.

Dies hat den Vorteil, dass jede Führungshülse zur Aufnahme einer weiteren, inneren Führungshülse oder zur Aufnahme eines Bohr- bzw. Fräswerkzeugs verwendbar ist.

In einer überdies bevorzugten Ausgestaltung der Neuerung ist vorgesehen, dass mindestens eine Führungshülse an ihrem oberen Ende einen radial nach außen gerichteten trichterförmigen Kragen aufweist.

Aus fertigungstechnischen Gründen wird es bevorzugt, dass jede Führungshülse mit einem trichterförmigen Kragen ausgebildet ist, wobei dieser Kragen in der Art einer Montagehilfe für eine weitere, einzusteckende Führungshülse ausgebildet ist.

Es kann jedoch auch vorgesehen sein, dass die außen liegende Hülse anstatt des überstehenden Kragens nur eine kleine Anschrägung, etwa in Form einer Fase aufweist, um in ihrem Außendurchmesser möglichst schmal ausgebildet zu sein.

Darüber hinaus ist die trichterförmige Ausbildung des Kragens als Einführungshilfe für einen Dentalbohrer bzw. eines Dentalfräswerkzeuges zu verwenden.

Für den Fall, dass die Außenhülse nur eine Anschrägung in Form einer Fase aufweist wird die Einführhilfe, welche auch als Tiefenanschlag für ein Werkzeug dienen kann, durch die erste eingesetzte Innenhülse bereitgestellt, an welcher der erfindungsgemäße Kragen ausgebildet ist.

In einer bevorzugten Ausgestaltung der Neuerung ist vorgesehen, dass das Bohr- bzw. Fräswerkzeug am Aufnahmeschaft einen Zentrierkonus aufweist.

Dieser Zentrierkonus bildet im Zusammenspiel mit dem trichterförmigen Kragen einer angeordneten Führungshülse eine zusätzliche Zentrierung für das Werkzeug in einem zahnmedizinischen Bohrführungssystem aus.

Darüber hinaus kann dieser Zentrierkonus in der Art eines Tiefenanschlages verwendet werden, wodurch eine aufwendige Kontrolle der erforderlichen Bohrtiefe für das Implantat entfällt.

In einer überdies bevorzugten Ausgestaltung der Neuerung ist vorgesehen, dass die Haltevorrichtung zur Aufnahme der Führungsvorrichtung eine Ausnehmung aufweist, welche dem Außendurchmesser der aufzunehmenden Führungsvorrichtung angepasst ist.

Diese angepasste Ausnehmung in der Haltevorrichtung bildet eine formschlüssige Verbindung aus, die zusätzlich die axiale Ausrichtung der Führungshülsen in der Haltevorrichtung unterstützt.

Überdies wird in einer weiteren Ausgestaltung der Neuerung vorgesehen, dass die Haltevorrichtung Mittel zur Positionierung, insbesondere Vorsprünge und Vertiefungen zur Definition der Bohr- bzw. Fräsrichtung auf einem zu behandelnden Kieferbereich umfasst.

Die Ausbildung von Vorsprüngen und Vertiefungen hat den Vorteil, dass mit der neuerungsgemäßen Haltevorrichtung eine passgenaue Auflagefläche der Haltevorrichtung auf dem Kieferbereich bzw. auf der Oberfläche eines Zahnkörpers möglich ist, wodurch eine gezielte Ausrichtung des einzubringenden Implantats gewährleistet werden kann.

Es ist vorgesehen, dass die Führungshülse auf ihrem Außenumfang ringförmige Vertiefungen aufweist.

Derartige Vertiefungen sind in der Art einer Sollschnittstelle ausgeführt, die eine flexible und einfache Anpassung einer Führungshülsenlänge ausbildet, indem eine Verkürzung der Führungshülse beispielsweise durch Abtrennen eines ringförmigen Segmentes ausführbar ist.

Darüber hinaus bilden die ringförmigen Vertiefungen einen verbesserten Kontakt der Führungshülse in der Haltevorrichtung aus, wenn diese mittels eines Verbindungsmittels, beispielsweise Zement oder dergleichen, mit der Haltevorrichtung verbunden wird.

Weitere Merkmale der Neuerung gehen aus der nachfolgenden Figurenbeschreibung und den Zeichnungen hervor.

Dabei zeigen:
- Figur 1: eine Gesamtansicht eines zahnmedizinischen Bohrführungssystems im Einsatz;
- Figur 2: eine Führungsvorrichtung aus einzelnen, ineinander gesteckten Führungshülsen;
- Figur 3: eine perspektivische Ansicht einer inneren Führungshülse;
- Figur 4: eine perspektivische Ansicht einer äußeren Führungshülse;
- Figur 5: eine perspektivische Ansicht eines Fräs- bzw. Bohrwerkzeugs.
- Figur 6: eine Darstellung einer Außenhülse und einer Innenhülse.

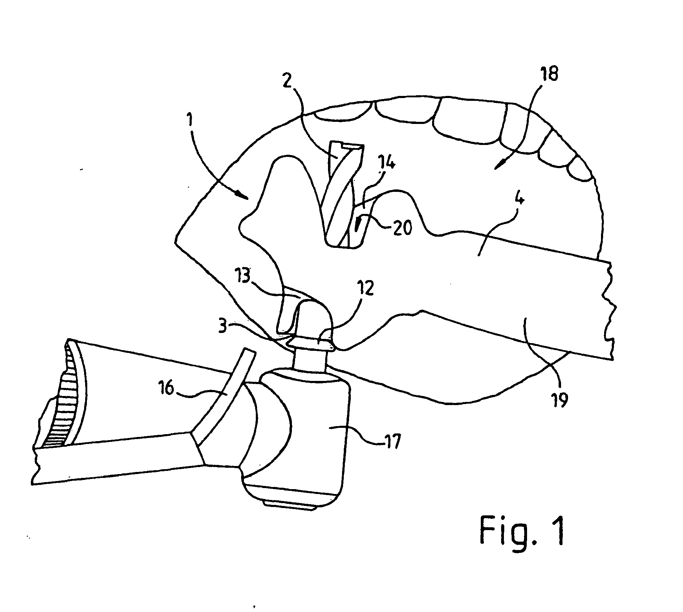

In Figur 1 wird ein neuerungsgemäßes Bohrführungssystem 1 gezeigt, welches eine Haltevorrichtung 4, eine von der Haltevorrichtung 4 aufgenommene Führungsvorrichtung 3 und ein Bohr- bzw. Fräswerkzeug 2 umfasst.

Für einen drehenden Antrieb wird das Bohr- bzw. Fräswerkzeug 2 von einer Bohr- bzw. Fräsvorrichtung 17 angetrieben. Während der Behandlung wird vorzugsweise das Bohr- bzw. Fräswerkzeug 2 sowie die herzustellende Kieferbohrung mittels einem Reinigungs- bzw. Kühlmittel frei von Bohrpartikeln gespült und gleichzeitig gekühlt. Die Zufuhr des Kühlmittels erfolgt über eine Kühlmittelzufuhr 16, die an der Bohr- bzw. Fräsvorrichtung 17 entsprechend befestigt ist.

Die Haltevorrichtung 4 weist an ihrem Ende in einer nahezu senkrechten Anordnung mit Bezug auf einen einstückig angeformten Haltegriff 19 Vorsprünge 13 bzw. Vertiefungen 14 auf, welcher der Kontur der Kieferoberfläche bzw. der Kontur eines Zahnkörpers angepasst ist.
Das Ende an der Haltevorrichtung 4 kann beidseitig mit entsprechenden Vorsprüngen 13 bzw. Vertiefungen 14 ausgebildet sein.

Das Ende der Haltevorrichtung 4 ist zusätzlich von einer Ausnehmung 20 durchsetzt, die in einer nahezu senkrechten Anordnung zur axialen Verlängerung des Haltergriffs 19 angeordnet ist. Diese Ausnehmung 20 ist zur Aufnahme der Führungsvorrichtung 3 angepasst.

Zur Herstellung einer Kieferbohrung wird das Bohr- bzw. Fräswerkzeug 2 in die Führungsvorrichtung 3 eingeführt, wobei zeitgleich die Haltevorrichtung 4 mit ihren Vorsprüngen 13 bzw, Vertiefungen 14 auf der Kontur einer Kieferoberfläche bzw, auf der Kontur eines Zahnkörpers aufliegt.

Das Bohr- bzw. Fräswerkzeug 2 weist auf seiner äußeren Mantelfläche einen Zentrierkonus 12 auf, welcher die axiale Führung in der Führungsvorrichtung 3 unterstützt und einen Tiefenanschlag für eine herzustellende Kieferbohrung ausbildet.

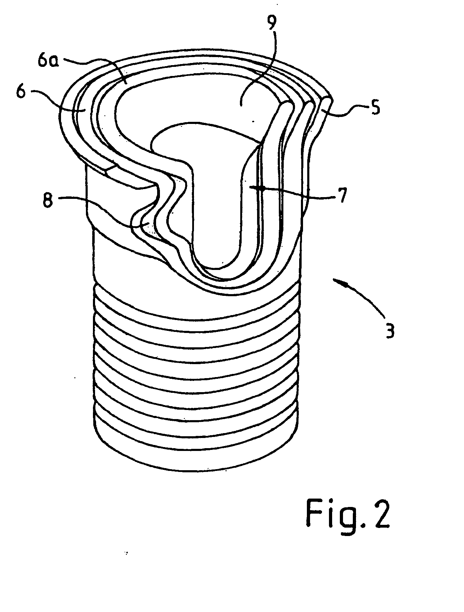

In Figur 2 wird eine Führungsvorrichtung 3 gezeigt, wobei beispielsweise in einer äußeren Führungshülse 5 mindestens eine, vorzugsweise zwei innere Führungshülsen 6 und 6a eingesteckt sind.

Dabei weisen die äußere und innere Führungshülsen 5, 6, 6a an ihrer radial außen liegenden Mantelfläche teilweise eine sich axial erstreckende Durchbrechung 7 auf, die in einen radial nach außen gerichteten, trichterförmigen Kragen 9 übergeht.

Der trichterförmige Kragen 9 ist derart ausgebildet, dass eine innere Führungshülse 6a auf die Innenoberfläche eines trichterförmigen Kragens 9 einer äußeren Führungshülse 6 passt. Zusätzlich weist die innere Führungshülse 6a auf ihrer äußeren Mantelfläche eine radial nach außen gewölbte Auswölbung 8 auf, welche im ineinander gesteckten Zustand zweier Führungshülsen 6, 6a in einer gegenüberliegend angeordneten Auswölbung 8 der jeweils außen liegenden Führungshülse 6 eingreift. Die Verbindung der Hülsen gegen ein längsaxiales Auseinanderziehen erfolgt nach der Art eines Bajonettverschlusses, welcher aufgrund der ineinander greifenden Auswölbungen fixiert ist. An ihren rückseitigen Enden können die Auswölbungen der inneren Hülsen bzw. die Ausnehmung der äußeren Hülse eine ineinander greifende Form aufweisen, welche jedoch aufgrund auftretender Drehmomente verklemmen kann. Verbessert (jedoch vorliegend nicht dargestellt) werden die rückseitigen Enden als ebene stumpf stoßende Flächen ausgebildet, welche sich nicht ineinander verkeilen und dadurch verklemmen können.

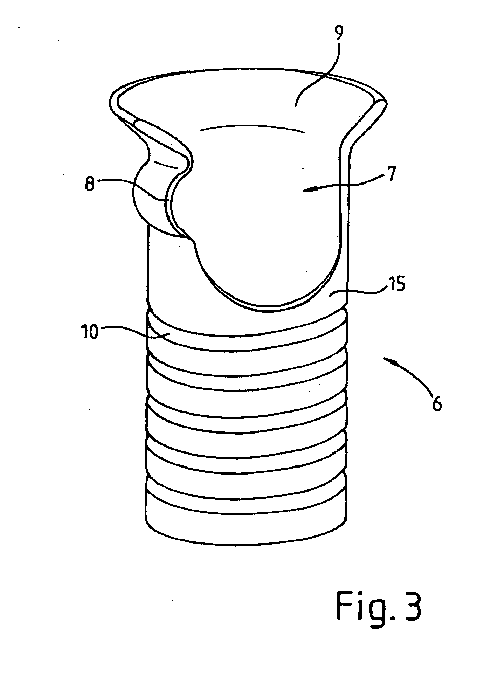

In Figur 3 wird eine perspektivische Einzeldarstellung einer inneren Führungshülse 6, 6a gezeigt, welche an ihrem oberen Ende den trichterförmigen Kragen 9 aufweist und eine mindestens teilweise sich auf der Manteloberfläche erstreckende Auswölbung 8 aufweist. Die Auswölbung 8 ist im hier gezeigten Ausführungsbeispiel unterhalb des trichterförmigen Kragens 9 angeordnet.

Zusätzlich ist die Mantelfläche der inneren Führungshülse 6 im oberen Bereich in axialer Richtung teilweise durchbrochen.

Diese Durchbrechung 7 gewährleistet ein Einführen einer weiteren inneren Führungshülse 6a, die mit ihrer entsprechenden Auswölbung 8 in dem Bereich der Durchbrechung 7 der hier gezeigten Führungshülse 6 eingeführt wird. Anschließend werden die beiden Auswölbungen 8 der ineinander gesteckten Führungshülsen 6, 6a durch Verdrehen der beiden Führungshülsen 6, 6a in entgegen gesetzter Drehrichtung miteinander in Eingriff gebracht.
Diese lösbare Verbindung von zwei ineinander gesteckten Führungshülsen 6, 6a vermeidet eine unerwünschte Verschiebung der Führungshülsen 6, 6a in axialer Richtung sowie ein so genanntes "Mitdrehen" einer eingesteckten Führungshülse 6, 6a beim Herstellen einer Kieferbohrung.

Auf der nach unten weisenden Manteloberfläche weist die Führungshülse 5, 6, 6a radial umlaufende Vertiefungen 10 auf, welche in der Art einer Sollschnittstelle zu verwenden sind. Aufgrund dieser Vertiefungen 10 kann die Länge der Führungshülse 6 durch ein vereinfachtes Abtrennen eines Mantelrings gekürzt werden.

Darüber hinaus bilden diese Vertiefungen 10 auf der Manteloberfläche einer äußeren Führungshülse 5 verbesserte Verbindungseigenschaften in einer entsprechend zugeordneten Ausnehmung der Haltevorrichtung 4 aus, sollte beispielsweise die Verbindung zwischen Führungshülse 5 und Haltevorrichtung 4 mittels einem Haftmittel, beispielsweise Zement hergestellt werden.

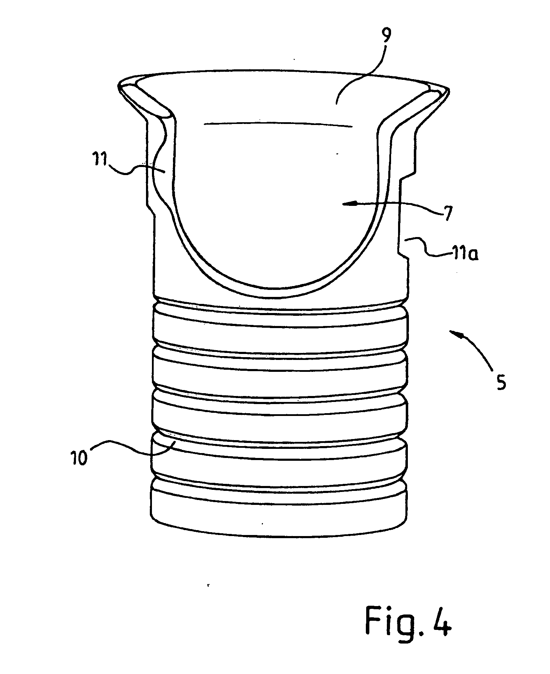

In Figur 4 ist eine perspektivische Ansicht einer äußeren Führungshülse 5 gezeigt, deren rotationssymmetrischen Ausformungen sowie der Durchmesser an die Abmessungen einer aufzunehmenden Führungshülse 6, 6a angepasst sind.

Dabei ist es vorteilhaft, dass die äußere Führungshülse 5 eine teilweise radial umlaufende Ausnehmung 11 aufweist, die auf der Innenoberfläche unterhalb des trichterförmigen Kragens 9 ausgebildet ist und mit der Auswölbung 8 einer einzusteckenden Führungshülse 6 im Eingriff zu bringen ist.
Aus fertigungstechnischen Gründen ist es durchaus möglich, dass die äußere Führungshülse 5 ebenfalls eine Auswölbung auf ihrer äußeren Manteloberfläche aufweist, welche einer Auswölbung 8 einer inneren Führungshülse 6 entspricht.

Zusätzlich kann eine Kontaktfläche 11a zur Verbesserung der Anbringung in der Haltevorrichtung 4 vorgesehen sein.

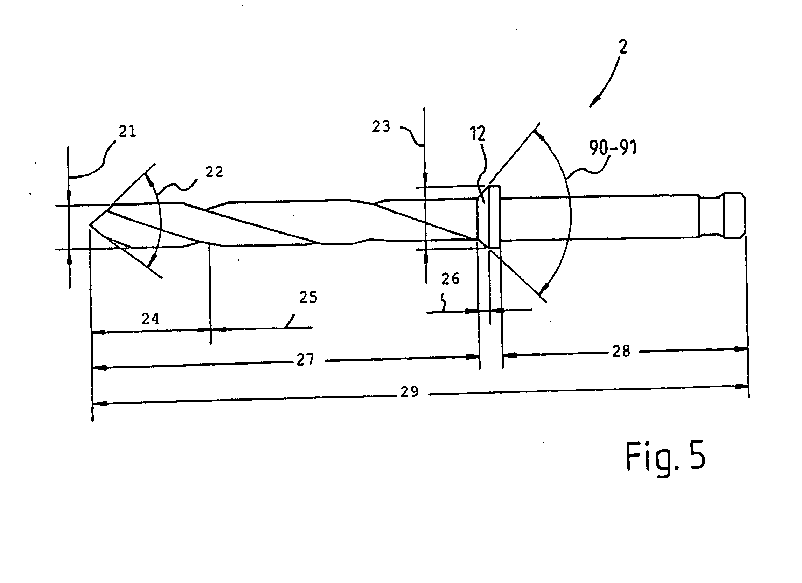

In Figur 5 ist ein Bohr- bzw. Fräswerkzeug 2 gezeigt, welches oberhalb einer Wendelnut einen radial nach außen gerichteten Zentrierkonus 12 aufweist. Vorzugsweise weist das Werkzeug 2 im Verlauf der Wendelnut eine Führungsfase auf, die im hinteren Bereich gebrochen ist, um eine reibverminderte Rotation des Werkzeugs 2 in der Führungshülse 5 auszubilden.

Der Zentrierkonus 12 bildet im Zusammenspiel mit dem trichterförmigen Kragen 9 einer Führungshülse 5, 6, 6a eine zusätzliche, axiale Führung des Fräs- bzw. Bohrwerkzeugs 2 aus.
Darüber hinaus bildet der Zentrierkonus 12 im Zusammenspiel mit dem trichterförmigen Kragen 9 einen Tiefenanschlag für das Bohr- bzw. Fräswerkzeug 2 bei der Herstellung einer Kieferbohrung aus.

Dabei ist es möglich, dass der Zentrierkonus 12 einstückig mit dem Bohr- bzw. Fräswerkzeug 2 oder als separater Konusring ausgebildet ist. Als separater Konusring kann dieser mit einem entsprechenden Befestigungsmittel auf dem Werkzeugschaft befestigt werden. Dadurch kann ein variabel einstellbarer Tiefenanschlag für das Werkzeug gebildet werden.

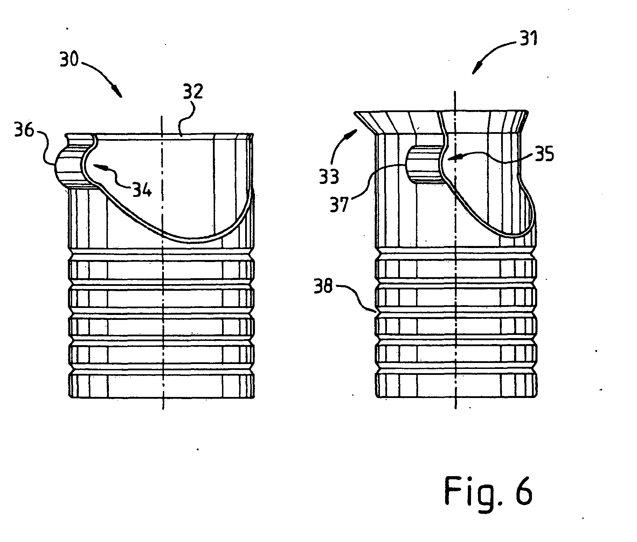

In Figur 6 ist eine Ausführung einer erfindungsgemäßen Außenhülse 30 sowie einer Innenhülse 31 dargestellt. Die Außenhülse 30 ist ohne einen Kragen am oberen Rand ausgeführt, so dass nur eine kleine Fase 32 zur verbesserten Einführbarkeit der Innenhülse vorgesehen ist. Der als Führung und Tiefenanschlag für ein Werkzeug notwendige Kragen 33 wird erst an der Innenhülse 31 ausgebildet, so dass der Platzbedarf der Außenhülse 30 minimiert wird.

Die Außenhülse 30 sowie die Innenhülse 31 weisen in ihren Mantelflächen Ausnehmungen 34 sowie Auswölbungen 35 auf, welche in ineinander gestecktem Zustand eine Verbindung nach der Art eines Bajonett-Verschlusses bilden. Am Ende 36 der Ausnehmung 34 ist diese als im Wesentlichen stumpfes Ende ausgeformt, welches eine quasi eine Ebene bildet. Die Auswölbung 35 weist entsprechend ebenfalls ein stumpfes ebenes Ende 37 auf, so dass diese Enden in formschlüssig ineinander greifendem Zustand der Ausnehmung 34 und der Auswölbung 35 flächig aufeinander stoßen, und ein Verklemmen vermieden wird.

Erfindungsgemäß sind die Hülsen auf ihrer Außenseite mit Längsrillen 38 versehen, welche unter Anderem auch als Schnitthilfen zur definierten Abtrennung auf geforderte Längen eingesetzt werden.

### Bezugszeichenliste:

- 1: Bohrführungssystem
- 2: Bohr- bzw. Fräswerkzeug
- 3: Führungsvorrichtung
- 4: Haltevorrichtung
- 5: Äußere Führungshülse
- 6: Innere Führungshülse
- 7: Durchbrechung
- 8: Auswölbung
- 9: Kragen, trichterförmig
- 10: Vertiefung, ringförmig
- 11: Ausnehmung
- 11a: Kontaktfläche
- 12: Zentrierkonus
- 13: Vorsprung
- 14: Vertiefung
- 15: Mantelfläche
- 16: Kühlmittelzufuhr
- 17: Bohr- bzw. Fräsvorrichtung
- 18: Mundraum
- 19: Haltegriff
- 20: Ausnehmung, Haltevorrichtung 4
- 30: Außenhülse
- 31: Innenhülse
- 32: Fase
- 33: Kragen
- 34: Ausnehmung
- 35: Auswölbung
- 36: Ende der Ausnehmung
- 37: Ende der Auswölbung
- 38: Längsrillen

## Patentansprüche

1. Zahnmedizinisches Bohrführungssystem (1) für eine zahnmedizinische Implantatbehandlung, umfassend ein Bohr- bzw. Fräswerkzeug (2), eine Führungsvorrichtung (3) zur axialen Führung des Bohr- bzw. Fräswerkzeugs (2) und eine Haltevorrichtung (4) zur Aufnahme der Führungsvorrichtung, wobei die Führungsvorrichtung (3) aus mindestens zwei ineinander steckbaren, separaten Führungshülsen (5, 6, 6a) gebildet ist wobei mindestens eine Führungshülse (5, 6, 6a) auf ihrem Außenumfang ringförmige Vertiefungen (10) aufweist
**dadurch gekennzeichnet, dass**
die Mantelfläche jeder Führungshülse (5, 6, 6a) in axialer Richtung teilweise durchbrochen ist.

2. System nach Anspruch 1, **dadurch gekennzeichnet, dass** die mindestens zwei ineinander steckbaren Führungshülsen (5, 6, 6a) lösbar miteinander verbunden sind.

3. System nach einem der vorhergehenden Ansprüche 1 bis 2, **dadurch gekennzeichnet, dass** die Verbindung der Führungshülsen (6, 6a) mittels einer auf der Manteloberfläche jeder inneren Führungshülse (6, 6a) teilweise angeordneten, radial nach außen gerichteten Auswölbung (8) ausgebildet wird.

4. System nach einem der vorhergehenden Ansprüche 1 bis 3, **dadurch gekennzeichnet, dass** die im direkten Kontakt mit der Haltevorrichtung (4) stehende Führungshülse (5) auf ihrer inneren Mantelfläche eine Ausnehmung (11) aufweist.

5. System nach einem der vorhergehenden Ansprüche 1 bis 4, **dadurch gekennzeichnet, dass** eine radial nach außen gerichtete Auswölbung (8) einer inneren Führungshülse (6, 6a) in eine Ausnehmung (11) in der Mantelfläche einer die innere Führungshülse (6, 6a) aufnehmenden äußeren Führungshülse (5) mittels Formschluss der Auswölbung (8) in der Ausnehmung (11), vorzugsweise mit einem stumpfen, insbesondere an ebenen Ausnehmungsenden in Mantelumlaufrichtung ausgebildeten Anschlag, eingreift.

6. System nach einem der vorhergehenden Ansprüche 1 bis 5, **dadurch gekennzeichnet, dass** die innere Führungshülse (6, 6a) einen Innendurchmesser aufweist, welcher einem Außendurchmesser des zu führenden Bohr- bzw. Fräswerkzeugs (2) angepasst ist.

7. System nach einem der vorhergehenden Ansprüche 1 bis 6, **dadurch gekennzeichnet, dass** mindestens eine Führungshülse (5, 6, 6a) an ihrem oberen Ende einen radial nach außen gerichteten, trichterförmigen Kragen (9) aufweist.

8. System nach einem der vorhergehenden Ansprüche 1 bis 7 **dadurch gekennzeichnet, dass** das Bohr- bzw. Fräswerkzeug (2) am Aufnahmeschaft einen Zentrierkonus (12) aufweist.

9. System nach einem der vorhergehenden Ansprüche 1 bis 8, **dadurch gekennzeichnet, dass** die Haltevorrichtung (4) zur Aufnahme der Führungsvorrichtung (3) eine Ausnehmung (20) aufweist, welche dem Außendurchmesser der aufzunehmenden Führungsvorrichtung (3) angepasst ist.

10. System nach einem der vorhergehenden Ansprüche 1 bis 9, **dadurch gekennzeichnet, dass** die Haltevorrichtung (4) Mittel zur Positionierung, insbesondere Vorsprünge (13) und Vertiefungen (14) zur Definition der Bohr- bzw. Fräsrichtung auf einem zu behandelnden Kieferbereich umfasst.

## Claims

1. A dental drilling guidance system (1) for a dental implant treatment, comprising a drilling- or milling tool (2), a guidance device (3) for the axial guidance of the drilling- or milling tool (2) and a holding device (4) for accommodating the guidance device, wherein the guidance device (3) is formed from at least two separate guidance sleeves (5, 6, 6a) which can be plugged into each other, wherein at least one guidance sleeve (5, 6, 6a) has ring-shaped indentations (10) on its outer circumference **characterized in that**
the lateral area of each guidance sleeve (5, 6, 6a) is partially broken away in the axial direction.

2. A system according to claim 1, **characterized in that** the at least two guidance sleeves (5, 6, 6a) which can be plugged into each other are detachably connected to each other.

3. A system according to one of the preceding claims 1 to 2, **characterized in that** the connection of the guidance sleeves (6, 6a) is formed by means of a protrusion (8) directed radially outward, which is in part arranged on the lateral surface area of each inner guidance sleeve (6, 6a).

4. A system according to one of the preceding claims 1 to 3, **characterized in that** the guidance sleeve (5) in direct contact with the holding device (4) has a recess (11) on its inner lateral area.

5. A system according to one of the preceding claims 1 to 4, **characterized in that** a protrusion (8) of an inner guidance sleeve (6, 6a) directed radially outward engages in a recess (11) in the lateral area of an outer guidance sleeve (5) receiving the inner guidance sleeve (6, 6a) by means of positive form locking of the protrusion (8) in the recess (11), preferably with a blunt end stop formed particularly on flat recess ends in the direction of rotation of the lateral surface.

6. A system according to one of the preceding claims 1 to 5, **characterized in that** the inner guidance sleeve (6, 6a) has an inner diameter, which is adjusted to an outer diameter of the drilling or milling tool (2) to be guided.

7. A system according to one of the preceding claims 1 to 6, **characterized in that** at least one guidance sleeve (5, 6, 6a) has a funnel-shaped collar (9) directed radially outward on its upper end.

8. A system according to one of the preceding claims 1 to 7, **characterized in that** the drilling- or milling tool (2) has a centering cone (12) on the mounting shank.

9. A system according to one of the preceding claims 1 to 8, **characterized in that** the holding device (4) for accommodating the guidance device (3) has a recess (20), which is adjusted to the outer diameter of the guidance device (3) to be accommodated.

10. A system according to one of the preceding claims 1 to 9, **characterized in that** the holding device (4) comprises means for the positioning, particularly projections (13) and indentations (14) for defining the drilling- or milling direction in a jaw area to be treated.

## Revendications

1. Système de guidage pour perçage (1) en médecine dentaire pour un traitement d'implant dentaire, comportant un outil de perçage et/ou de fraisage (2), un dispositif de guidage (3) permettant le guidage axial de l'outil de perçage et/ou de fraisage (2) et un dispositif de fixation (4) destiné à recevoir le dispositif de guidage, ledit dispositif de guidage (3) étant constitué par au moins deux manchons de guidage (5, 6, 6a) séparés, enfichables l'un dans l'autre, au moins l'un desdits manchons de guidage (5, 6, 6a) comportant des creux (10) annulaires sur sa périphérie extérieure, **caractérisé en ce que** la paroi latérale de chacun des manchons de guidage (5, 6, 6a) est percée partiellement dans le sens axial.

2. Système selon la revendication 1, **caractérisé en ce que** lesdits au moins deux manchons de guidage (5, 6, 6a) enfichables l'un dans l'autre sont assemblés entre eux de manière amovible.

3. Système selon l'une quelconque des revendications précédentes 1 à 2, **caractérisé en ce que** l'assemblage des manchons de guidage (6, 6a) est réalisé au moyen d'un bombement (8) dirigé radialement vers l'extérieur, disposé en partie sur la surface de la paroi latérale de chacun des manchons de guidage (6, 6a) intérieurs.

4. Système selon l'une quelconque des revendications précédentes 1 à 3, **caractérisé en ce que** le manchon de guidage (5) en contact direct avec le dispositif de fixation (4) comporte un évidement (11) sur sa paroi latérale intérieure.

5. Système selon l'une quelconque des revendications précédentes 1 à 4, **caractérisé en ce qu'**un bombement (8), dirigé radialement vers l'extérieur, d'un manchon de guidage (6, 6a) intérieur s'engage dans un évidement (11) dans la paroi latérale d'un manchon de guidage (5) extérieur logeant le manchon de guidage (6, 6a) intérieur, au moyen d'un assemblage par conjugaison de forme du bombement (8) dans l'évidement (11), de préférence avec une butée émoussée, réalisée en particulier sur les extrémités planes de l'évidement dans le sens du pourtour de la paroi latérale.

6. Système selon l'une quelconque des revendications précédentes 1 à 5, **caractérisé en ce que** le manchon de guidage (6, 6a) intérieur possède un diamètre intérieur qui est ajusté au diamètre extérieur de l'outil de perçage et/ou de fraisage (2) à être guidé.

7. Système selon l'une quelconque des revendications précédentes 1 à 6, **caractérisé en ce qu'**au moins un manchon de guidage (5, 6, 6a) comporte, au niveau de son extrémité supérieure, un collet (9) en forme de trémie, orienté radialement vers l'extérieur.

8. Système selon l'une quelconque des revendications précédentes 1 à 7, **caractérisé en ce que** l'outil de perçage et/ou de fraisage (2) comporte un cône de centrage (12) sur sa tige de réception.

9. Système selon l'une quelconque des revendications précédentes 1 à 8, **caractérisé en ce que** le dispositif de fixation (4) comporte, pour recevoir le dispositif de guidage (3), un évidement (20) qui est ajusté au diamètre extérieur du dispositif de guidage (3) à être reçu.

10. Système selon l'une quelconque des revendications précédentes 1 à 9, **caractérisé en ce que** le dispositif de fixation (4) comporte des moyens de positionnement, en particulier des saillies (13) et des creux (14), permettant de définir la direction de perçage et/ou de fraisage sur une zone à traiter du maxillaire.
